Europäisches Patentamt

European Patent Office

Office européen des brevets

(19)

(11) Numéro de publication : **0 007 339**
**B1**

(12) **FASCICULE DE BREVET EUROPÉEN**

(45) Date de publication du fascicule du brevet :
**19.05.82**

(51) Int. Cl.³ : **A 61 B  6/14**

(21) Numéro de dépôt : **78900060.1**

(22) Date de dépôt : **18.07.78**

(86) Numéro de dépôt international :
**PCT/FR 78/00011**

(87) Numéro de publication internationale :
**WO WO/79000 (22.02.79 Gazettee 79/04)**

(54) **APPAREIL POUR RADIOGRAPHIE PANORAMIQUE.**

(30) Priorité : **26.07.77 FR 7722885**

(43) Date de publication de la demande :
**06.02.80 (Bulletin 80/03)**

(45) Mention de la délivrance du brevet :
**19.05.82 Bulletin 82/20**

(84) Etats contractants désignés :
**DE FR GB SE**

(56) Documents cités :
**BE - A - 351 443**
**FR - A - 2 022 715**
**US - A - 1 876 069**
**US - A - 3 237 309**
**US - A - 3 636 349**
**US - A - 3 737 660**
**US - A - 3 806 732**

(73) Titulaire : **THOMSON-CSF**
**173, Boulevard Haussmann**
**F-75360 Paris Cedex 08 (FR)**

(72) Inventeur : **PALLUET, Jean Noel**
**Compagnie Gén. de radiologie 13 square Max Hymans**
**F-75741 Paris Cedex 15 (FR)**

(74) Mandataire : **Thrierr, Françoise et al**
**THOMSON-CSF SCPI 173, Bld Haussmann**
**F-75360 Paris Cedex 08 (FR)**

Imprimerie Jouve, 18, rue St-Denis, 75001 Paris, France

Appareil pour radiographie panoramique

L'invention concerne un appareil pour radiographie panoramique d'une surface courbe, notamment de l'arcade dentaire.

Un tel appareil se compose essentiellement d'un statif supportant un bras oscillant auquel sont suspendus la source de rayons et le porte-film. Le bras se déplace autour de la surface à radiographie en décrivant une courbe aussi voisine que possible de la surface dont on veut obtenir l'image. Pratiquement, pour être convenablement adaptée à la forme elliptique de l'arcade dentaire, il est souhaitable que cette courbe soit une ellipse dont on ajuste autant que possible l'allongement à la forme de la mâchoire à radiographier ; si tel n'était pas le cas on obtiendrait sur le film radiographique une projection déformée des différentes zones radiographiées.

Le brevet américain 3.636.349 délivré le 18 janvier 1972 décrit un appareil de radiographie panoramique dans lequel un bras supportant à une extrémité la source de rayons X, et à l'autre extrémité un porte-film, se déplace autour de la tête du patient selon deux mouvements combinés. Le bras est pour cela accroché à un axe vertical qui d'une part se déplace sur une courbe approchant la forme de la mâchoire, et qui, d'autre part tourne sur lui-même. Le faisceau collimaté de rayons X frappe ainsi les différents points de la mâchoire fixe, la direction du faisceau étant variable par rapport à un repère fixe.

Ce mouvement est obtenu, dans une première version où la courbe décrite par l'arbre supportant le bras mobile est une partie de cercle, en fixant cet arbre au centre d'un pignon denté satellite engrenant à l'intérieur d'une couronne dentée fixe dont le diamètre est voisin du double de celui du pignon satellite.

Un moto-réducteur entraîne en rotation sur elle-même une pièce qui entraîne elle-même l'arbre précipité dans un déplacement circulaire autour du centre de la couronne dentée. Lorsque l'arbre se déplace ainsi sur un demi-cercle, il entraîne le pignon satellite qui roule sans glisser dans la couronne et tourne environ d'un tour complet puisque le rapport des diamètres est voisin de deux.

L'arbre entraîne alors le bras porte-source et porte-film qui se déplace autour de l'arcade dentaire et tourne sur lui-même. Il est à noter que le sens de déplacement de l'arbre sur la portion de cercle qu'il décrit et le sens de rotation de cet arbre sur lui-même sont inverses. Il est encore à noter que, l'arbre tournant sur lui-même d'une quantité qui lui est imposée par la rotation du pignon satellite dans la couronne, le bras qui en est solidaire, et donc le faisceau de rayons X, sont loin d'être toujours orthogonaux à la mâchoire dans la zone frappée par les rayons.

Dans une deuxième version de cet appareil, le bras porte-source, porte-film est monté sur l'arbre actionné comme précédemment décrit, par l'intermédiaire d'une pièce d'excentration à excentration réglable. Lorsque l'arbre se déplace sur son cercle en tournant sur lui-même comme décrit ci-dessus, l'axe supportant le bras décrit une portion d'ellipse dont la courbure peut être réglée par l'excentration précitée. Le déplacement du bras peut ainsi mieux épouser la forme de la mâchoire ; mais la non-orthogonalité du faisceau de rayons X par rapport à la mâchoire subsiste. La projection des différentes zones radiographiées sur le film radiographique se fait donc différemment, et l'image panoramique de la mâchoire sur le film, est déformée.

Un objet de l'invention est de réaliser un appareil permettant d'obtenir un cliché panoramique de la mâchoire sans déformation ni flou, ce qui est possible si le bras porte-tube et porte-film est constamment perpendiculaire à l'arcade dentaire.

Pour cela, dans un appareil conforme à l'invention, qui comporte un bras porte-source et porte-film monté sur un arbre qu'un mécanisme à pignon satellite engrenant dans une roue dentée fait se déplacer sur une courbe elliptique, le bras est monté fou sur l'arbre qui l'entraîne et peut tourner autour de cet arbre qui constitue pour lui un axe de rotation. Ainsi cet arbre entraîne le bras oscillant dans son mouvement elliptique sans lui imposer pour autant une direction fixe. La direction du bras est donnée par un dispositif d'entraînement auxiliaire qui l'entraîne en rotation autour de son axe de rotation de telle manière qu'il soit toujours normal à la tangente à l'ellipse décrite par ledit axe de rotation. Pour cela le bras oscillant est couplé au dispositif à pignon satellite par ce dispositif d'entraînement auxiliaire.

Une des causes de déformation de la projection étant ainsi supprimée par cette combinaison des deux dispositifs d'entraînement du bras qui l'obligent à toujours rester orthogonal à l'ellipse que décrit son axe de rotation, d'autres sources de déformation du cliché panoramique sont supprimées ou réduites par l'appareil de l'invention. Les autres sources de déformation possible de l'image panoramique projetée sont liées au déroulement du film sur lequel se projette l'image radiographique au fur et à mesure de son élaboration. En effet, on aura, dans le sens de la hauteur des dents, sur l'image projetée, une déformation constante égale au grandissement dû à la projection ; ce grandissement est fonction du rapport entre les distances foyer-film et foyer-arcade dentaire. Il est donc souhaitable, pour éviter des déformations, d'obtenir un grandissement équivalent de l'arcade développée dans le sens de sa longueur, le grandissement, dans ce sens, dépendant notamment du déplacement du film dans son support.

Il faut donc que la longueur de l'arcade dentaire sur le film soit fonction de la longueur d'ellipse décrite par l'arbre entraînant le bras, au facteur de grandissement près, et que la vitesse de défilement du film derrière la fente du porte-film soit proportionnelle à la vitesse d'avancement de cet arbre sur sa trajectoire elliptique. Or, le porte-film est fixé sur le bras qui, en plus du déplacement elliptique que lui fait subir l'arbre qui l'entraîne est animé d'un mouvement de rotation autour de ce dernier. L'appareil de l'invention comporte un mécanime d'entraînement du film qui permet que la vitesse de déroulement du film soit proportionnelle à la vitesse de translation de l'arbre sur son ellipse.

L'appareil de l'invention permet encore d'obtenir un cliché radiographique de bonne qualité grâce à un mécanisme intercalé entre le moto-réducteur et le dispositif à pignon satellite qui permet de faire déplacer l'arbre entraînant le bras à une vitesse variable sur l'ellipse qu'il décrit, pour compenser les variations d'absorption des rayons X, notamment lorsque le faisceau doit traverser la colonne vertébrale.

D'autres objets, caractéristiques et résultats de l'invention ressortiront de la description suivante donnée à titre d'exemple non limitatif et illustrée par les figures annexées qui représentent :

la figure 1, une vue schématique, en perspective, d'un mode de réalisation d'un appareil de radiographie panoramique conforme à l'invention ;

les figures 2 et 3, des courbes permettant de comprendre le mode de fonctionnement d'un tel appareil ;

les figures 4a, 4b et 4c, des courbes illustrant différentes formes de l'ellipse obtenue ;

la figure 5, une vue partielle, en plan, du dispositif de défilement du film ;

la figure 6, une vue en plan, d'un mode de réalisation possible du mécanisme d'entraînement du dispositif à pignon satellite.

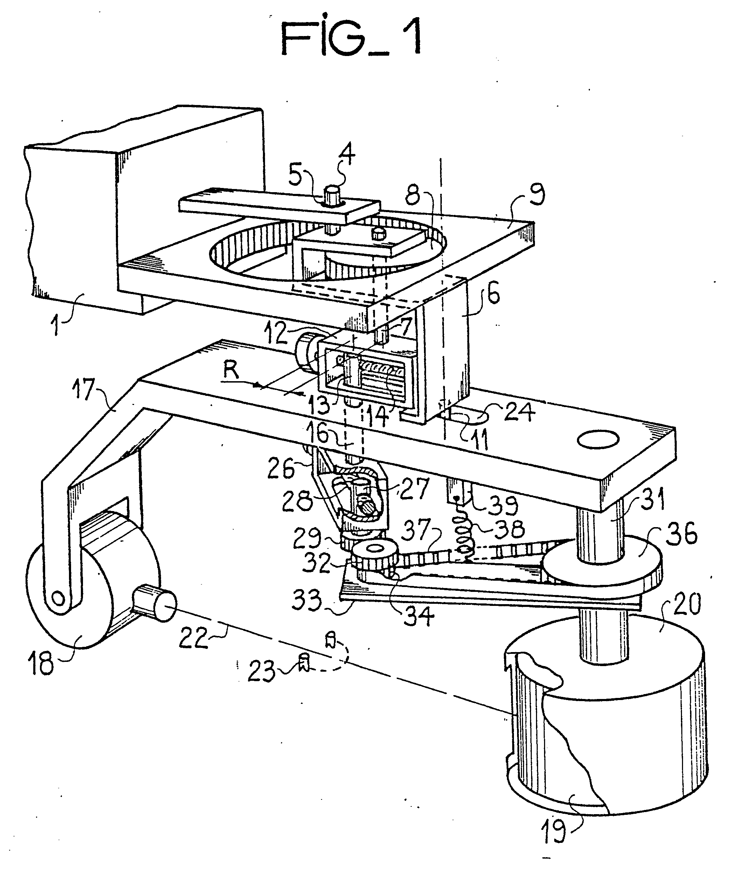

On voit sur la figure 1 en 1 le bâti du statif reposant sur le sol par une partie non représentée. Un groupe moto-réducteur non représenté entraîne en rotation un arbre primaire 4 tourillonant dans un palier 5 solidaire du bâti 1. Cet arbre primaire 4 est solidaire d'une pièce 6 en forme de S. Dans cette pièce 6 tourillonne un arbre secondaire 7 sur lequel est fixé un pignon denté 8 qui engrène à l'intérieur d'une couronne dentée 9 solidaire du bâti 1. Le diamètre du pignon denté 8 est la moitié du diamètre de la couronne dentée 9. La pièce 6 en forme de S porte un ergot 11 dont il sera question plus loin. L'extrémité de l'arbre secondaire 7 porte à son extrémité inférieure un bloc support ou porte-coulisseau 12. Ce porte-coulisseau 12 comporte, sur sa face inférieure, une rainure dans laquelle peut se déplacer un coulisseau constitué par un écrou 13 manœuvré par une vis d'excentration 14. Tandis que l'écrou 13 est parallèle aux arbres primaires et secondaire, parallèles entre eux, la vis 14 leur est perpendiculaire. Elle permet de faire varier la distance R entre l'arbre secondaire 7 et l'écrou 13 qui se prolonge, à son extrémité inférieure, par un arbre tertiaire 16 parallèle aux arbres primaire et secondaire.

Il est possible, dans une version simplifiée de l'appareil de l'invention, de remplacer le porte-coulisseau 12 et son coulisseau réglable par une liaison fixe entre les deux axes 7 et 16 ; dans ce cas on aura R = constante.

Le bras oscillant 17 est monté fou sur l'arbre tertiaire 16 ; il peut ainsi tourillonner librement autour de cet arbre tertiaire qui constitue pour lui un axe de rotation. Ce bras 17 comporte une rainure 24, sensiblement parallèle au faisceau de rayons X 22. L'ergot 11 mentionné plus haut est engagé dans cette rainure 24.

Le bras oscillant 17 porte à une de ses extrémités une source de rayons X 18, et à son autre extrémité une boîte 20 porte-film (en forme de tambour, par exemple).

La source de rayons X émet un faisceau 22 en direction d'une fente aménagée dans la boîte 20, en traversant l'arcade dentaire 23 d'un patient positionné à cet effet par un dispositif connu, non représenté.

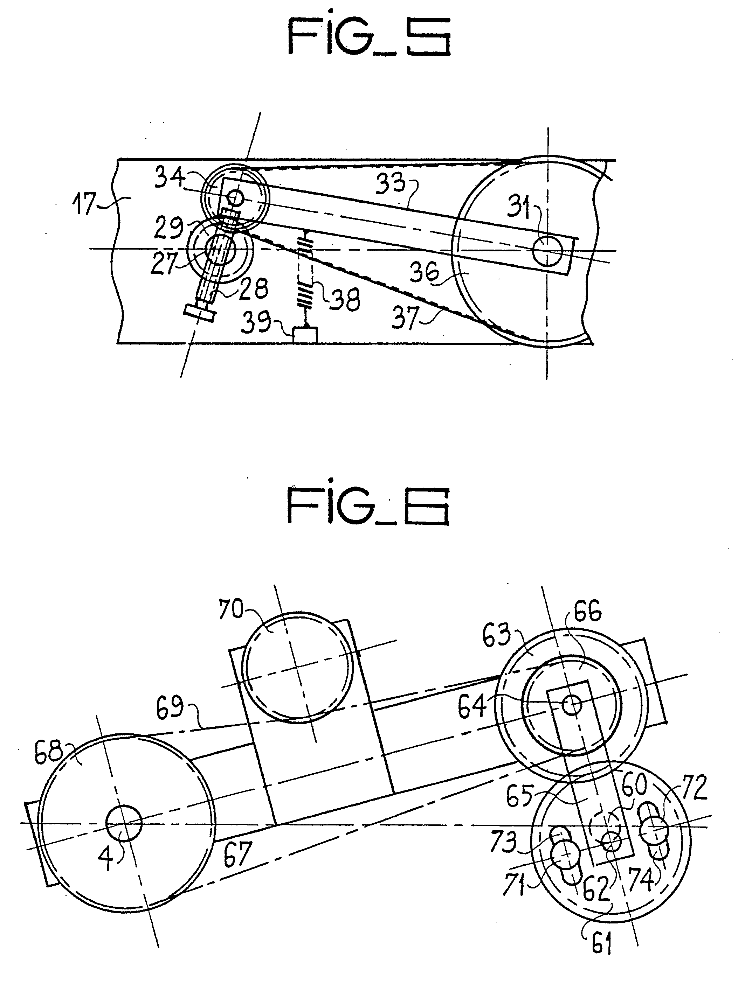

Le film 19 est entraîné dans la boîte 20 de manière à défiler devant une fente de la boîte laissant passer les rayons X. Le dispositif d'entraînement du film 19 sera décrit en détail à l'aide de la figure 5. Il est à noter que ce dispositif entraîne le tambour support de film 19, mais pas la boîte 20. Cette boîte est fixée directement au bras 17 lui-même, par un dispositif non représenté. Elle ne tourne pas avec le tambour, la fente devant être toujours en face de la source de rayons X.

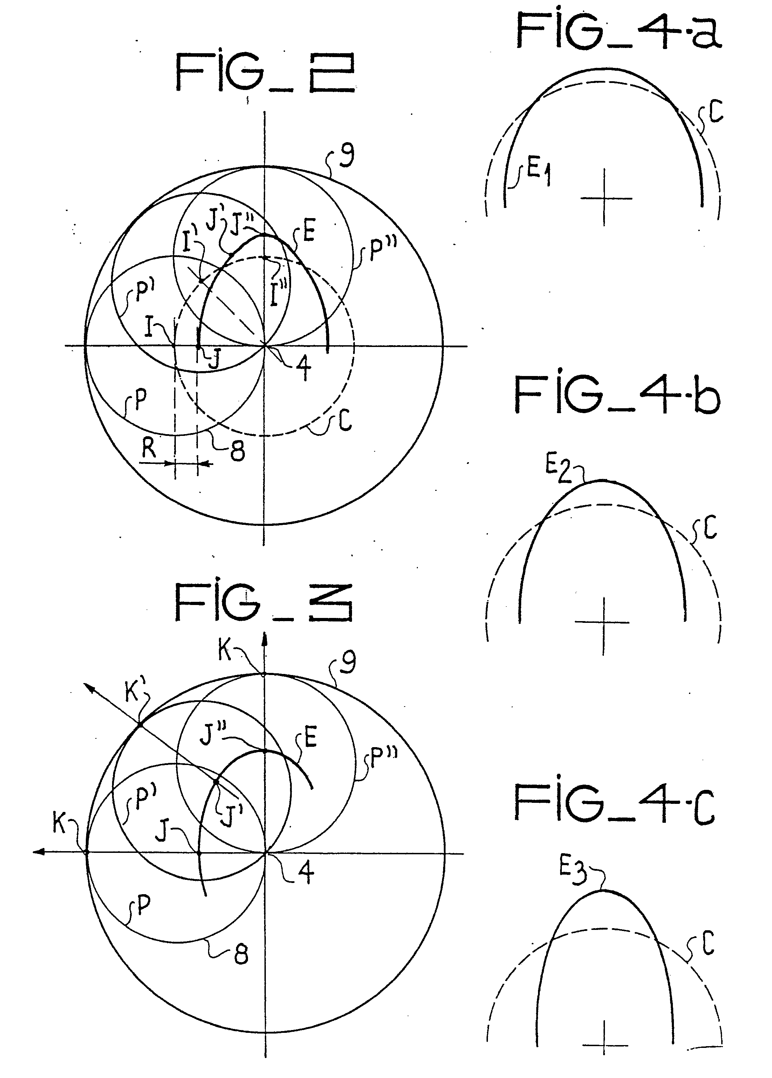

Le fonctionnement de l'appareil de l'invention va maintenant être décrit, à l'aide notamment des figures 2, 3 et 4.

Lorsque l'arbre primaire 4 est entraîné en rotation par le moto-réducteur, la pièce en S 6 tourne avec lui et entraîne l'arbre secondaire 7 dont l'axe décrit un cercle autour de l'arbre primaire 4. On a représenté sur la figure 2, par leurs diamètres primitifs, la couronne dentée 9 et le pignon denté 8 de diamètre moitié, vus de dessus. Le centre de la couronne dentée 9 correspond à l'axe de l'arbre primaire 4 ; le centre I du pignon denté 8 correspond à l'axe de l'arbre secondaire 7. Le point I décrit donc un cercle C autour du point 4. Lorsque l'arbre secondaire 7 tourne ainsi autour de 4, le pigon denté 8 engrène à l'intérieur de la roue dentée 9, venant par exemple d'une première position P à une deuxième P', puis à une troisième P'' etc... Son centre I (axe de l'arbre secondaire) passe respectivement de I à I', puis à I'' sur le cercle C.

Pendant que le pignon denté 8 roule ainsi (sans glisser) sur la couronne 9, un point de sa circonférence (correspondant à son diamètre primitif) décrit une droite, et un point quelconque J situé entre son centre I et cette circonférence, décrit une ellipse. Si le point J considéré se trouve à une distance R de I, la trajectoire J', J'' etc... qu'il parcourt est une ellipse E de grand axe D + 2R et de petit axe D-2R (D étant le diamètre primitif du pignon 8).

Le point J est l'axe tertiaire 16 qui décrit ainsi une ellipse dont la courbure est déterminée par le réglage de la distance R = IJ, c'est-à-dire par le réglage de la vis d'excentration 14. Le réglage de cette vis permet d'obtenir : un cercle pour R = 0 (cercle C), et des ellipses de plus en plus allongées au fur et à mesure que R augmente. La figure 4 indique des allures de telles ellipses $E_1$, $E_2$, $E_3$ par rapport au cercle C obtenu pour R = 0.

Il est ainsi aisé d'adapter la forme de l'ellipse à la forme de la mâchoire à radiographier. Dans la version simplifiée précipitée où R n'est pas réglable, on choisit une valeur de R correspondant à une mâchoire standard.

Le fonctionnement du mécanisme qui vient d'être décrit entraîne le déplacement de l'arbre tertiaire 16 selon une ellipse (dont le plan est perpendiculaire audit arbre). Un mécanisme complémentaire, combiné à celui-ci va permettre, grâce au fait que le bras 17 est monté fou sur l'arbre tertiaire, que ce bras soit normal à la tangente à l'ellipse pour toutes les positions de l'arbre 16. Ce mécanisme comporte la rainure 24, parallèle à l'axe du bras, et l'ergot 11 solidaire de la pièce 6 en S ; cet ergot 11 est positionné, sur la pièce 6, à l'aplomb du point de tangence de la couronne 9 et du pignon 8. La figure 3 permet de comprendre comment cet ergot et cette rainure, combinés au mécanisme d'entraînement du bras selon une ellipse, assurent cette fonction.

L'ergot 11, à l'aplomb du point de tangence de la couronne 9 et du pignon 8, se trouve (dans la vue en plan de la figure 3) dans les différentes positions K, K' et K" lorsque l'arbre tertiaire 16 décrit l'ellipse E (l'axe de cet arbre étant projeté en J, J' et J"). Or les droites KJ, K'J' et K"J" sont toujours normales aux tangentes à l'ellipse E respectivement en J. J' et J".

Ainsi l'ergot 11 qui, lorsqu'il coulisse dans la rainure 24 du bras 17, se trouve toujours sur l'axe longitudinal de ce bras, maintient cet axe confondu avec les droites KJ, K'J', K"J" etc... et maintient le bras orthogonal à la tangente à l'ellipse. Le faisceau de rayons 22 est ainsi toujours perpendiculaire à la portion de mâchoire qu'il traverse.

La figure 5 montre en plan une vue de dessous à une échelle plus grande que celle de la figure 1 du mécanisme de déroulement du film.

Ce mécanisme comporte (figure 1) un bloc support 26, ou porte-coulisseau, fixé à l'extrémité inférieure de l'arbre tertiaire 16. Un coulisseau, traversant une fente située sur la face inférieure de ce porte-coulisseau et constitué d'un écrou 27, peut être déplacé dans son porte-coulisseau par la manœuvre d'une vis d'excentration 28. Cette vis 28 est située dans un plan perpendiculaire aux axes des arbres précédents. L'écrou 27 est solidaire d'un pignon denté 29 qui tourne donc avec l'arbre tertiaire 16 tout en étant excentré par rapport à lui, d'une distance réglable par la vis d'excentration 28.

Un levier 33, pouvant tourner autour de l'arbre 31, lui même tourillonnant dans le bras 17, porte à son autre extrémité un couple pignon denté 32 — roue crantée 34. Le pignon denté 32 est en prise avec le pignon 29 tandis qu'une courroie crantée 37 relie les deux parties crantées 34 et 36. Un ressort 38 (figure 5) tendu entre le levier 33 et une pièce 39 descendant du bras 37, tire le levier 33 vers l'axe du bras, ce qui permet au pignon denté 32 d'être en prise avec le pignon 29 quelle que soit l'excentricité de l'axe du pignon 29 par rapport à l'arbre 16.

Ce dispositif de défilement du film permet, ainsi qu'il a été dit plus haut, de maintenir un grandissement constant du cliché, indépendamment de la courbure de l'ellipse décrite ; il permet ceci en faisant défiler le film 19 derrière la fente du porte-film 29 à une vitesse proportionnelle à la vitesse d'avancement de l'arbre tertiaire 16 sur sa trajectoire elliptique.

Il résulte de la disposition illustrée sur la figure 5 que le porte-film 20 est entraîné en rotation par le pignon 29 par l'intermédiaire des pignon-poulies crantées 34 et 36. Ce mouvement de rotation est la somme de deux mouvements. Un premier mouvement de rotation du pignon 29 entraîné par l'arbre tertiaire 16, et un mouvement de l'axe du pignon 29 par rapport à l'axe tertiaire 16 d'oscillation du bras 17. Ce deuxième mouvement est fonction de l'excentricité affichée par la vis d'excentration 28. Il est adapté à la courbure de l'ellipse décrite par l'axe de l'arbre 16, en excentrant le pignon 29 à l'aide de la vis 28 d'une valeur proportionnelle à l'excentration R donnée par la vis 14. La proportionnalité entre les exentricités est fonction des rapports existant entre les diamètres des poulies et pignons de 29 à 36. Le bon grandissement sur les clichés est obtenu par le choix judicieux desdits rapports. Une fois ces rapports choisis, on établit une correspondance de l'excentricité à réaliser par la vis 28 en fonction de celle adoptée pour la vis 14, lors de l'ajustage de l'ellipse sur l'arc dentaire. Il est à noter que si la distance R est constante, l'axe tertiaire 18 étant relié à l'axe secondaire 7 par une pièce fixe comme déjà signalé, l'excentration du pignon 29 par rapport à l'axe 16 peut également être constante et la fixation assurée par une pièce non réglable.

Ainsi qu'il a déjà été dit, il est souhaitable que l'ellipse E ne soit pas décrite à vitesse constante par l'arbre tertiaire 16 qui entraîne le bras, pour que le cliché ne soit pas sous-exposé dans la zone des incisives où le faisceau de rayon X traverse la zone dense que constitue la colonne cervicale avant de venir frapper les incisives, puis le film.

Une solution de certains appareils de l'art antérieur consiste à corriger ce défaut en augmentant la pénétration des rayons X, donc la haute tension de la source, lors du passage dans cette région.

Dans l'appareil de l'invention, cette correction se fait automatique et uniquement mécaniquement.

Il est intéressant, avant d'en venir au mécanisme de correction proprement dit pour éviter les effets néfastes de la colonne cervicale, de noter une propriété de l'appareil de l'invention.

En envisageant, en l'absence de ce mécanisme de correction, que l'arbre primaire 4, et donc le pignon denté 8, tournent à vitesse constante, l'appareil réalise déjà une pré-correction. En effet lorsque le pignon C tourne à vitesse constante dans la roue 9, le point J (figure 3) se déplace moins vite dans la zone de forte courbure de l'ellipse E. Mais cette pré-correction, intrinsèquement liée au mode d'obtention du mouvement du bras selon l'invention, peut s'avérer insuffisante pour compenser l'effet de la colonne cervicale, et ce d'autant plus qu'elle est réduite par un contre effet. Si le point J se déplace plus lentement dans la zone de forte courbure, la normale (K'J', K"J" etc...) à l'ellipse tourne plus vite dans cette zone de forte courbure. On a donc un déplacement elliptique du bras plus lent, mais une rotation de ce même bras, autour de son axe de rotation (arbre tertiaire 16) plus rapide.

Le dispositif de correction qui permet d'obtenir un ralentissement (réglable) de la vitesse de déplacement sur l'ellipse au passage des incisives, consiste à modifier la vitesse de rotation de l'arbre primaire 4 de façon non uniforme.

La figure 6 montre, vu de dessus, un moyen d'obtenir, avec l'appareil de l'invention, un cliché qui ne soit pas affaibli dans la zone des incisives, grâce à une vitesse variable de rotation de l'arbre primaire 4. Cette vitesse variable de l'arbre 4 est obtenue par l'introduction, entre l'arbre 60 du moto-réducteur et l'arbre primaire 4, d'un dispositif constitué de la manière suivante. Sur l'axe 60 du moto-réducteur est monté un pignon denté 61 dont l'axe géométrique 62 peut être excentré de façon réglable par rapport à l'axe 60 du moto-réducteur. Ce pignon 61 engrène avec un pignon denté 63 de même diamètre que lui, et qui tourne librement autour d'un axe 64. Cet axe 64 est relié à l'axe géométrique 62 du pignon 61 par une biellette 65 de longueur égale au diamètre primitif des pignons 61 et 63. Grâce à cette biellette, l'engrènement des deux pignons est toujours assuré ; elle est de plus montée libre sur les axes 62 et 64.

Un pignon à chaîne 66 est monté coaxialement au pignon 63 dont il est solidaire. Ce pignon à chaîne 66 tourne librement par rapport à l'axe 64, tout comme le pignon 63.

L'axe 64 est fixé sur une deuxième biellette 67 qui peut tourner autour de l'arbre 4 qui est l'arbre primaire de l'appareil de l'invention.

Sur cet arbre 4 est fixé, solidairement, un pignon à chaîne 68 possédant un nombre de dents double du pignon 63. Le pignon 63 entraîne le pignon 68 en rotation par une chaîne 69 tendue par un galet 70 monté sur la biellette 67.

L'excentration du pignon 61 par rapport à l'axe 60 du moto-réducteur qui entraîne le tout peut être réglée par exemple au moyen de vis 71 et 72 solidaires de l'arbre 60 du moto-réducteur, ces vis passant dans des boutonnières 73 et 74 du pignon 61. L'excentration dépendra de la position de ces vis dans les boutonnières.

Le fonctionnement de ce dispositif est le suivant.

Si l'excentration est nulle, les axes 60 et 62 étant coaxiaux, les vitesses de rotation des différents pignons sont constantes puisque le moto-réducteur tourne à vitesse constante ; les biellettes sont immobiles et l'arbre 4 tourne à une vitesse moitié de l'axe 60 du moto-réducteur.

Si par contre on excentre l'axe 62 par rapport à l'axe 60 du moto-réducteur, on introduit une variation de vitesse de rotation de l'arbre primaire 4. En effet, tandis que le pignon 61 tourne à vitesse constante, le pignon 63 et le pignon 66 qui en est solidaire tournent à vitesse variable avec un maximum et un minimum de vitesse espacés d'un demi-tour. La biellette 67 oscille autour de l'axe 4 qui constitue le centre du système.

La réduction étant de 1/2 entre les pignons 66 et 68, l'arbre 4, entraîné par le pignon 68 tourne à une vitesse variable, mais avec deux maxima et deux minima par tour.

En choisissant convenablement l'excentration entre les axes 60 et 62 on peut programmer un ralentissement plus ou moins important dans le déplacement elliptique du bras 17 porte-source et porte-film, au moment de son passage au droit des incisives du patient. Le choix de cette excentration peut par exemple être fait en fonction de l'homogénéité de noircissement des clichés panoramiques pour la moyenne des patients.

Il peut encore être noté qu'un effet intéressant résulte de la combinaison des deux causes de variations de vitesse du déplacement de l'arbre tertiaire 16 sur sa trajection elliptique, l'une étant la variation de vitesse imposée à l'arbre primaire 4 par un dispositif approprié tel que décrit, l'autre étant la variation inhérente au mécanisme de production de l'ellipse (tel que décrit plus haut). En effet, à cause de cette combinaison, les minima et maxima de vitesse du bras ne sont pas espacés de 90° mais d'un angle fonction de l'excentration du pignon 61. Il en résulte le fonctionnement suivant : le bras démarre dans une zone de petite vitesse puis accélère ; il ralentit de nouveau dans la zone des incisives, réaccélère et termine son mouvement en petite vitesse.

Ce fonctionnement présente l'avantage de faire démarrer le bras en petite vitesse avec un moteur asynchrone, donc à vitesse constante ce qui permet d'éliminer les oscillations du départ, visibles sur les clichés.

**Revendications**

1. Appareil pour radiographie panoramique comportant, sur un bâti (1), un dispositif moto-réducteur entraînant un arbre primaire (4) en rotation sur lui-même, cet arbre (4) entraînant à son tour, en rotation autour de lui, un arbre secondaire (7) qui lui est parallèle et qui porte un pignon denté (8) satellite engrenant, lorsque l'arbre secondaire se déplace, à l'intérieur d'une couronne dentée (9) fixe, un arbre tertiaire (16), parallèle aux deux autres, étant fixé à l'arbre secondaire de manière à en être distant d'une distance R prédéterminée, de manière que l'arbre tertiaire (16) se déplace sur une ellipse (E) lorsque l'arbre primaire (4) est actionné, cet arbre tertiaire (16) supportant un bras oscillant (17) muni à l'une de ses extrémités d'une source de rayons X (18) émettant un faisceau collimaté (22) en direction d'un porte-film (20) fixé à l'autre extrémité du bras, le porte-film (20) comportant une fente exposée au faisceau et un dispositif (26-39) d'entraînement du film (19) en fonction des déplacements du bras, appareil caractérisé en ce que :

— le diamètre de la couronne dentée fixe (9) est égale au double de celui du pignon satellite (8) de manière que lorsque l'arbre primaire (4) fait un tour complet sur lui-même, le pignon satellite (8) fasse deux tours dans la couronne fixe (9) ;

— le bras (17) est monté fou sur l'arbre tertiaire (16) qui l'entraîne ainsi dans son mouvement elliptique sans lui imposer pour autant une direction fixe ;

— le bras (17) est couplé à l'arbre primaire (4) par l'intermédiaire d'un dispositif (6, 11, 24) d'entraînement auxiliaire lié à cet arbre primaire et tel que, pour chaque position de l'arbre primaire, et donc pour chaque position de l'arbre tertiaire (16) sur l'ellipse (E), la direction du bras (17) est orthogonale à cette ellipse.

2. Appareil selon la revendication 1, caractérisé en ce que le dispositif (6, 11, 24) entraînement auxiliaire du bras (17) comporte des moyens qui déterminent la position du bras (17) autour de l'axe de rotation que constitue pour lui l'axe de l'arbre tertiaire (16) de telle manière qu'à chaque instant l'axe longitudinal du bras passe par ledit axe de rotation et par un point qui est à l'aplomb du point de tangence de la couronne fixe (9) et de son pignon satellite (8).

3. Appareil selon la revendication 2, caractérisé en ce que le dispositif (6, 11, 24) d'entraînement auxiliaire du bras (17) comporte un ergot (11), fixé sur une pièce (6) solidaire de l'arbre primaire (4) et situé dans un plan passant par les axes des arbres primaire (4) et secondaire (7), à une distance de l'arbre primaire (4) égale au diamètre du pignon satellite (8), cet ergot (11) coulissant dans une rainure (24) pratiquée dans le bras oscillant (17) selon son axe longitudinal, et sensiblement parallèle au faisceau (22) de rayons X qui est ainsi maintenu dans une position sensiblement orthogonale à l'ellipse (E) décrite par l'axe de l'arbre tertiaire en chacun de ses points.

4. Appareil selon l'une des revendications 1 à 3, caractérisé en ce que l'arbre tertiaire (16) est rendu solidaire de l'arbre secondaire (7) par un bloc-support (12) fixé à l'arbre secondaire et comportant un écrou (13) déplaçable perpendiculairement à l'arbre secondaire (7), cet écrou étant positionné par une vis d'excentration (14) et supportant l'arbre tertiaire, le réglage de la vis (14) permettant de régler la courbure de l'ellipse (E) sur laquelle se déplace l'arbre tertiaire (16).

5. Appareil selon l'une des revendications 1 à 4, caractérisé en ce que le dispositif (26-34) d'entraînement du film (19) dans son porte-film (20) comporte : un axe (31) tournant dans le bras (17) et sur lequel sont fixés le porte-film (20) et une première poulie crantée (36) ; un premier pignon dentée (29) fixé à l'arbre tertiaire par une pièce telle que ce pignon soit excentré par rapport à cet arbre ; un deuxième pignon denté (33) tournant autour de l'axe (31) supportant le porte-film (20) ; un ressort de rappel (38) assurant un engrènement permanent des deux dits pignons ; une courroie crantée (37) réunissant la poulie crantée (36) montée sur l'axe porte-film à une deuxième poulie crantée (34) solidaire du deuxième pignon denté (32).

6. Appareil selon les revendications 4 et 5, caractérisé en ce que le premier pignon denté (29) est fixé à l'arbre tertiaire (16) par un bloc-support (26) fixé à cet arbre tertiaire, le pignon denté (29) étant lui-même fixé à un écrou (27) se déplaçant, perpendiculairement à l'arbre tertiaire, par une vis d'excentration (18) dont le réglage dépend de celui de la vis d'excentration (14) qui détermine la courbure de l'ellipse (E) sur laquelle se déplace l'arbre tertiaire.

7. Appareil selon l'une des revendications précédentes, caractérisé en ce que l'arbre primaire (4) est couplé à l'arbre (60) du moto-réducteur tournant à vitesse constante, par un dispositif qui rend la vitesse de rotation de cet arbre primaire variable avec deux maxima et deux minima par tour d'arbre primaire, un minimum de vitesse se produisant lorsque l'arbre tertiaire (16) est à un sommet de l'ellipse E qu'il décrit.

8. Appareil selon la revendication 7, caractérisé en ce que le dispositif, intercalé entre l'arbre (60) du moto-réducteur et l'arbre primaire (4) comporte : un premier pignon denté (61) entraîné par l'arbre (60) du moto-réducteur dont il est excentré d'une quantité réglable ; un deuxième pignon denté (63) de même diamètre que le premier et maintenu engréné avec lui par une biellette (65), ce deuxième pignon (63) étant lui-même monté à une extrémité d'une biellette (67) qui peut tourner, à son autre extrémité, autour de l'axe primaire (4) ; un couple de pignons (66, 68) réducteurs de vitesse dans la proportion d'un à deux couplés par une chaîne (69), le premier pignon (66) de ce couple étant coaxial et solidaire du deuxième pignon denté précité (63), tandis que le deuxième pignon (68) de ce couple entraîne l'axe primaire (4).

## Claims

1. Panoramic radiographic apparatus comprising a reduction drive device on a frame (1), driving a primary shaft (4) rotating about itself, said sahft (4) driving a secondary shaft (7) rotating about itself which is parallel thereto and which holds a satellitepinion gear (8) meshing within a stationary gear ring (9) when the secondary shaft moves, a tertiary shaft (16) parallel to the two other shafts being secured to the secondary shaft in a manner to be spaced therefrom by a predetermined spacing R, so that the tertiary shaft (16) will move an ellipse (E) when the primary shaft (4) is operated, said tertiary shaft (16) supporting an oscillating arm (17) provided with an X-ray source (18) at one of its ends emitting a collimated beam (22) towards a film holder (20) secured on the other end of the arm, the film holder (20) comprising a slot exposed to the beam and a device (26-39) for driving the film (19) depending on the movements on the arm, the apparatus being characterized in that :
— the diameter of the stationary gear ring (9) is equal to twice the diameter of the satellite pinion (8) so that the satellite pinion (8) will perform two revolutions within the stationary ring (9) for one complete revolution of the primary shaft (4) about itself ;
— the arm (17) is mounted free on the tertiary shaft (16) which thus drives the same for its elliptic movement without imparting a fixed direction thereto ;
— the arm (17) is coupled to the primary shaft (4) through an auxiliary driving device (6, 11, 24) connected to the primary shaft and formed in such a manner that for each position of the primary shaft, thus for each position of the tertiary shaft (16) on the ellipse (E), the direction of the arm (17) is perpendicular to said ellipse.

2. Apparatus in accordance with claim 1, characterized in that the auxiliary driving device (6, 11, 24) of the arm (17) comprises means which determine the position of the arm (17) about the rotational axis formed therefor by the axis of the tertiary shaft (16) so that, at each moment, the longitudinal axis of the arm passes through that rotational axis and through a point vertically below the contacting point of the stationary ring (9) and its satellite pinion (8).

3. Apparatus in accordance with claim 2, characterized in that the auxiliary driving device (6, 11, 24) of the arm (17) comprises a stud (11) secured on a member (6) joined with the primary shaft (4) and lying in a plane passing through the axes of the primary (4) and the secondary (7) shafts at a distance from the primary shaft (4) equal to the diameter of the satellite pinion (8), said stud (11) sliding within a groove (24) arranged in the oscillating arm (17) along its longitudinal axis, and substantially parallel to the X-ray beam (22) which is thus held in a position substantially perpendicular to the ellipse (E) described by the axis of the tertiary shaft at each of its points.

4. Apparatus in accordance with any of claims 1 to 3, characterized in that the tertiary shaft (16) is joined with the secondary shaft (7) by a supporting block (12) secured to the secondary shaft and comprising a nut (13) movable perpendicularly to the secondary shaft (7), said nut (13) being positioned by an excenter screw (14) and supporting the tertiary shaft, the adjustment of the screw (14) allowing adjusement of the curvated of the ellipse (E) on which the tertiary shaft (16) is moved.

5. Apparatus in accordance with any of the claims 1-4, characterized in that the device (26-39) for driving the film (19) within its film holder (20) comprises : an axis (31) rotating in the arm (17) and on which the film holder (20) and a first ribbed pulley (36) are secured ; a first gear pinion (29) secured on the tertiary shaft by a member of such kind that the pinion is excentric with respect to said shaft ; a second gear pinion (32) meshing with the first (29) and mounted on a lever (33) rotating about the axid (31) supporting the film holder (20) ; a return spring (38) assuring permanent meshing of the two said pinions ; a toothed belt (37) connecting the ribbed pulley (36) mounted on the axis of the film holder to a second ribbed pulley (34) joined with the second gear pinion (32).

6. Apparatus in accordance with claims 4 and 5, characterized in that the first gear pinion (29) is secured on the tertiary shaft (16) through a supporting block (26) secured on said tertiary shaft, the gear pinion (29) being itself fixed to a nut (27) moved perpendiculary to the tertiary shaft by an excenter screw (18) the adjustment of which depends on that of the excenter screw (14) which determines the curvature of the ellipse (E) on which the tertiary shaft is moved.

7. Apparatus in accordance with any of the preceding claims characterized in that the primary shaft (4) is coupled to the shaft (60) of the reduction drive rotating at constant speed, by a device which renders the rotational speed of said primary shaft variable with two maxima and two minima per revolution of the primary shaft, a speed minimum occurring when the tertiary shaft (16) is at one apex of the ellipse (E) described thereby.

8. Apparatus in accordance with claim 7, characterized in that the device which is inserted between the shaft (60) of the reduction drive and the primary shaft (4) comprises : a first gear pinion (61) driven by the shaft (60) of the reduction drive with respect to which it is excentric by an ajustable amount ; a second gear pinion (63) of the same diameter as the first and maintained in meshing engagement therewith by a connecting-rod (65), said second pinion (63) being itself mounted on one end of a connecting rod (67) which may rotate about the primary shaft (4) at its other end ; a pair of speed reduction pinions (66, 68) of the ratio one by two and coupled by a chain (69), the first pinion (66) of said pair being coaxial with and joined to the second above mentioned gear pinion (63) whereas the second pinion (68) of said pair drives the primary shaft (4).

**Ansprüche**

1. Panorama-Radiographiegerät mit einer auf einem Gestell (1) angeordneten Motor-Untersetzungsgetriebeeinheit, welche eine Hauptwelle (4) in Drehung um sich selbst versetzt, wobei diese Welle (4) wiederum eine Nebenwelle (7) um sich selbst in Drehung versetzt, die zu ihr parallel ist und ein Satellitenritzel (8) trägt, das bei Bewegung der Nebenwelle mit einem feststehenden Zahnkranz (9) im Inneren desselben kämmt, wobei eine zu den beiden anderen Wellen parallele Tertiärwelle (16) an der Nebenwelle derart befestigt ist, daß sie von dieser einen vorbestimmten Abstand R aufweist, so daß die Tertiärwelle (16) sich auf einer Ellipse (E) bewegt, wenn die Hauptwelle (4) angetrieben wird, wobei diese Tertiärwelle (16) einen Schwingarm (17) trägt, der an seinem einen Ende eine Röntgenstrahlenquelle (18) trägt, die ein kollimiertes Bündel (22) in Richtung eines Filmträgers (20) aussendet, der am anderen Ende des Armes befestigt ist, wobei der Filmträger (20) einen von dem Bündel bestrahlten Schlitz und eine Vorrichtung (26-39) zum Antreiben des Filmes (19) in Abhängigkeit von den Bewegungen des Armes umfaßt, wobei das Gerät dadurch gekennzeichnet ist, daß :

— der Durchmesser des feststehenden Zahnkranzes (9) gleich dem doppelten Durchmesser des Satellitenritzels (8) ist, derart, daß, wenn die Hauptwelle (4) eine vollständige Drehung um sich selbst ausgeführt hat, das Satellitenritzel (8) zwei Umdrehungen in dem feststehenden Kranz (9) ausführt ;

— der Arm (17) auf der Tertiärwelle (16) frei gelagert ist, so daß diese Tertiärwelle ihn bei seiner elliptischen Bewegung antreibt, ohne ihm eine feste Richtung aufzuerlegen ;

— der Arm (17) an die Hauptwelle (4) über eine Hilfsantriebsvorrichtung (6, 11, 24) angekoppelt ist, die mit dieser Hauptwelle verbunden und so ausgebildet ist, daß für jede Stellung der Hauptwelle, also für jede Stellung der Tertiärwelle (16) auf der Ellipse (E), die Richtung des Armes (17) senkrecht zu dieser Ellipse ist.

2. Gerät nach Anspruch 1, dadurch gekennzeichnet, daß die Hilfsantriebsvorrichtung (6, 11, 24) des Armes (17) Mittel umfaßt, die die Stellung des Armes (17) um die Drehachse herum bestimmen, die für ihn durch die Achse der Tertiärwelle (16) gebildet ist, derart, daß zu jedem Zeitpunkt die Längsachse des Armes durch diese Drehachse und durch einen Punkt geht, der sich senkrecht unter dem Berührungspunkt des feststehenden Kranzes (9) mit seinem Satellitenritzel (8) befindet.

3. Gerät nach Anspruch 2, dadurch gekennzeichnet, daß die Hilfsantriebsvorrichtung (6, 11, 24) des Armes (17) einen Zapfen (11) umfaßt, der an einem mit der Hauptwelle (4) fest verbundenen Teil (6) befestigt ist und in einer Ebene liegt, die durch die Achsen der Hauptwelle (4) und der Nebenwelle (7) geht, und zwar in einem Abstand von der Hauptwelle (4), der gleich dem Durchmesser des Satellitenritzels (8) ist, wobei dieser Zapfen (11) in einer in dem Schwingarm (17) entlang deren Längsachse und im wesentlichen parallel zu dem Bündel (22) von Röntgenstrahlen angebrachten Rinne (24) gleitet, wobei das Bündel auf diese Weise in einer im wesentlichen senkrechten Stellung zu der Ellipse (E) gehalten wird, welche die Achse der Tertiärwelle an jedem ihrer Punkte beschreibt.

4. Gerät nach einem der Ansprüche 1-3, dadurch gekennzeichnet, daß die Tertiärwelle (16) mit der Nebenwelle (7) durch einen Trägerblock (12) fest verbunden ist, der an der Nebenwelle befestigt ist und eine Mutter (13) umfaßt, die senkrecht zur Nebenwelle (7) verscheibbar ist, wobei diese Mutter (13) durch eine Exzenterschraube (14) in Stellung gebracht wird und die Tertiärwelle trägt, wobei die Einstellung der Schraube (14) es ermöglicht, die Krümmung der Ellipse (E) einzustellen, auf der sich die Tertiärwelle (16) bewegt.

5. Gerät nach einem der Ansprüche 1-4, dadurch gekennzeichnet, daß die Vorrichtung (26-39) zum Antreiben des Films (19) in seinem Filmträger (20) umfaßt : eine Achse (31), die in dem Arm (17) drehbar ist und auf der der filmträger (20) und eine erste gerippte Riemenscheibe (36) befestigt sind ; ein erstes Zahnritzel (29), das an der Tertiärwelle über ein derart ausgebildetes Teil befestigt ist, daß das Ritzel in bezug auf diese Welle exzentrisch ist ; ein zweites Zahnritzel (32), das mit dem ersten (29) kämmt und an einem Hebel (33) gelagert ist, der sich um die den Filmträger (20) lagernde Achse (31) dreht ; wobei eine Rückholfeder (38) das dauernde Ineinanderkämmen der beiden Ritzel gewährleistet ; einen Zahnriemen (37), der die gerippte, auf der Achse des Filmträgers befestigte Riemenscheibe (36) mit einer zweiten gerippten Riemenscheibe (34) verbindet, die mit dem zweiten Zahnritzel (32) fest verbunden ist.

6. Gerät nach den Ansprüchen 4 und 5, dadurch gekennzeichnet, daß das erste Zahnritzel (29) an der Tertiärwelle (16) über einen Trägerblock (26) befestigt ist, der an dieser Tertiärwelle befestigt ist, wobei das Zahnritzel (29) selbst an einer Mutter (27) befestigt ist, die sich senkrecht zur Tertiärwelle und mittels einer Exzenterschraube (18) verschiebt, deren Einstellung von der der Exzenterschraube (14) abhängt, welche die Krümmung der Ellipse (E) bestimmt, auf der sich die Tertiärwelle bewegt.

7. Gerät nach einem der vorstehenden Ansprüche, dadurch gekennzeichnet, daß die Hauptwelle (4) an die Welle (60) der Motor-Untersetzungsgetriebeeinheit angekoppelt ist, die mit konstanter Geschwindigkeit rotiert, und zwar über eine Vorrichtung, welche die Rotationsgeschwindigkeit dieser Hauptwelle mit zwei Maxima und zwei Minima pro Umdrehung der Hauptwelle variabel macht, wobei ein Minimum der Geschwindigkeit auftritt, wenn die Tertiärwelle (16) sich an einem Scheitel der Ellipse (E) befindet, welche sie beschreibt.

8. Gerät nach Anspruch 7, dadurch gekennzeichnet, daß die zwischen die Welle (60) der Motor-Untersetzungsgetriebeeinheit und die Hauptwelle (4) eingefügte Vorrichtung umfaßt : ein erstes Zahnritzel (61), das durch die welle (60) der Motor-Untersetzungsgetriebeeinheit angetrieben wird, in bezug auf welche es um eine einstellbare Größe exzentrisch angeordnet ist ;

ein zweites Zahnritzel (63), das denselben Durchmesser wie das erste aufweist und mit diesem über eine kleine Pleuelstange (65) in Eingriff gehalten wird, wobei dieses zweite Ritzel (63) seinerseits an einem Ende einer kleinen Pleuelstange (67) gelagert ist, die an ihrem anderen Ende um die Hauptwelle (4) drehbar ist ; ein Untersetzungs-Ritzelpaar (66, 68), die eine Untersetzung im Verhältnis 1 zu 2 bewirken und durch eine Kette (69) gekoppelt sind, wobei das erste Ritzel (66) dieses Paares koaxial zu und fest verbunden mit dem zweiten zuvor genannten Zahnritzel (63) ist, während das zweite Ritzel (68) dieses Paares die Hauptwelle (4) antreibt.

# FIG_1

FIG_2

FIG_3

FIG_4·a

FIG_4·b

FIG_4·c

# FIG_5

# FIG_6